# EUROPEAN PATENT APPLICATION

(11) **EP 2 572 671 A1**
(43) Date of publication of application: **27.03.2013**
(21) Application number: 12006721.0
(22) Date of filing: 26.09.2012
(51) Int. Cl.: A61C 1/08, A61C 5/02, A61C 8/00

(54) **Stopper ring for a bone drill**

(30) Priority: 26.09.2011 JP 2011209171
(71) Applicant: GC Corporation, Bunkyo-ku Tokyo 113-0033 (JP)
(72) Inventor: Yoshihara, Hirotaka, Tokyo (JP); Fujii, Naoto, Tokyo (JP)
(74) Representative: Müller-Boré & Partner Patentanwälte

(57) **Abstract**

To provide a stopper enabling to easily and certainly regulate an entering depth of a blade of a drill in a jawbone of a lost tooth part by only inserting and attaching from the blade side of the drill which has a flange Xa projecting at a shank Xb, regardless of a size of a diameter of the blade.

The stopper Y is cylindrically formed in its entirety and inserted and attached from a top side of the blade Xc of the drill X which has the flange Xa projecting at the shank Xb. The stopper has a through hole to be penetrated by the blade Xc of the drill X and includes a drill blade entering depth regulating part Ya and a drill holding part Yb. The drill blade entering depth regulating part Ya has a height, regulating an entering depth of the blade Xc of the drill X in the jawbone of the lost tooth part. The drill holding part Yb has a plurality of slits Yba formed along the axial direction of the stopper from an end opposite to the drill blade entering depth regulating part Ya, detachably engages to the flange Xa of the drill X, and holds the drill X by elasticity.

## Description

The present invention relates to a stopper and its set for a dental drill. When boring a dental implant fixture embedding hole in a jawbone of a lost tooth part, the stopper is attached at the drill, in which a flange is projected at a shank of the drill, and regulates a depth of a blade of the drill entering in the jawbone of the lost tooth part, so that a depth of the dental implant fixture embedding hole is controlled.

A dental implant treatment has been carried out as one of dental prosthesis treatments, for recovering a oral function of the lost tooth part. The dental implant treatment is carried out by boring the dental implant fixture embedding hole in a jawbone of the lost tooth part, embedding a dental implant fixture made of titanium or a titanium alloy, which is superior in biocompatibility, so as to implant, and fixing the dental prosthesis to the dental implant fixture through an abutment.

In the dental implant treatment carried out in such a way, the work for boring the dental implant fixture embedding hole is carried out to bore the jawbone of the lost tooth part by using a dental driving device, such as a dental hand peace, or the like, in which a drill is attached.

However, the form of the jawbone and positions of nerves and blood vessels are different in each patient, so that it may be generated that the jawbone is broken or the nerves and blood vessels are damaged, when the dental implant fixture embedding hole is not bored at the desired position. Further, when the dental implant fixture embedding hole is not bored in the desired depth, there may be a case that a dental implant fixture embedded in the dental implant fixture embedding hole can not be firmly implanted or the dental prosthesis can not be stably fixed to the implanted dental implant fixture. Therefore, it is extremely important that bore the dental implant fixture embedding hole at a precise position and with a precise depth.

Thus, in recent years, a dentist takes various fault planes of the patient's jawbone using a dental CT before treatment, with the taken image data, grasps the form of the jawbone and positions of nerves and blood vessels, and then determines the embedding position and direction. By this way, a simulation of the completion state of the prosthesis device containing the dental implant fixture is performed.

Then, a surgical guide is made based on the data performing the simulation. The surgical guide is a tool attached in an oral cavity and assisting to determine a position and a direction of the dental implant fixture embedding hole. In the surgical guide, a guide hole is formed for guiding an advancing direction of a drill at a position corresponding to the embedding direction of the dental implant fixture determined based on the data performing the simulation. Therefore, the dentist can bore the dental implant fixture embedding hole at the desired position and direction by attaching the surgical guide made based on the data performing the simulation and boring in the jawbone of the lost tooth part according to the direction of the guide hole formed in the surgical guide.

On the other hand, for boring the dental implant fixture embedding hole with a desired depth, it has been conventionally performed that a stopper is attached to a drill, where the stopper regulates the depth in which a blade of the drill can enter the jawbone of the lost tooth part. As for the stopper, the stopper attached by using a screw, or the like, has been used. However, since the screw is used, there are problems that attaching/detaching thereof is time-consuming and the stopper can be moved if the attaching operation is not securely performed.

Further, there have been a drill in which a flange, which regulates the depth of entering of the blade to the jawbone of the lost tooth part, is integrally formed with a shank of the drill. When this drill is used, the depth of entering of the blade in the jawbone of the lost tooth part can be certainly regulated. However, since the flange is integrally projected with shank of the drill, there has been a problem that various drills must be prepared, where these drills are only different each other at the position of the flange in the axial direction (a length from the top end of the blade to the flange) even when they have the same form of the blade of the drill. For solving this problem, PCT Japanese Translation Patent Publication No. 2005-518834 discusses a ring regulating a entering depth of a blade of a drill in a jawbone of a lost tooth part. This ring is inserted from a top side of the blade of the drill, engages to the blade side part of the flange, and is fixed. If this ring is used, in a case where a form of a blade of a drill is the same, there is not necessary to prepare various drills which are different only at a position of a flange in an axial direction. However, in this ring, since the ring is engaged to the blade side part of the flange of the drill and fixed, the inner diameter of the ring is set to be smaller than the diameter of the blade side part of the flange of the drill. Thus, if the diameter of the blade side part of the flange of the drill is changed corresponding to the diameter of the embedded dental implant fixture, it is necessary to prepare various size rings which are different in only an inner diameter according to the diameter of the dental implant fixture, so that it is a problem.

Accordingly, the present invention is directed to provide a stopper which can regulate an entering depth of a blade of a drill in a jawbone of a lost tooth part by only inserting and attaching from the top of the blade side of the drill, regardless of a size of a diameter of the blade of the drill in which a flange is projected at a shank, and its stopper set.

Present inventors carried out earnest works to solve the aforementioned problems and, as a result, they found out the followings to complete the present invention. That is, a stopper is configured to be cylindrical shape in its entirety, where the stopper is attached to a drill having a flange projecting at a shank. The stopper has a through hole for being inserted by a blade of the drill. Further, the stopper includes a drill blade entering depth regulating part and a drill holding part. The drill blade entering depth regulating part has a height for regulating a entering depth of the blade of the drill in a jawbone of a lost tooth part. The drill holding part has a plurality of slits formed along an axial direction from an end opposite to the drill blade entering depth regulating part, engages detachably to the flange of the drill by elasticity, and holds the drill. In such a configuration, the stopper can be inserted from a top of the blade side of the drill since the stopper is configured to be cylindrical shape in its entirety, and can hold the drill since the drill holding part, which has a plurality of slits formed along the axial direction from the end opposite to the drill blade entering depth regulating part, engages detachably to the flange of the drill by elasticity. Thus, by unifying a diameter of the flange, the stopper can be attached detachably regardless of a diameter of the blade of the drill. Furthermore, when the stopper is attached to the drill, the drill blade entering depth regulating part can regulate the entering depth of the blade of the drill in the jawbone of the lost tooth part, so that the entering depth of the blade of the drill in the jawbone of the lost tooth part can be easily and certainly regulated.

That is, the present invention is a cylindrical stopper for regulating an entering depth of a blade of a drill to a jawbone of a lost tooth part. The drill is for boring a dental implant fixture embedding hole in the jawbone of the lost tooth part and has a flange. The stopper has a through hole for inserting a blade of the drill and includes a drill blade entering depth regulating part and a drill holding part. The drill blade entering depth regulating part has a height for regulating a entering depth in the jawbone of the lost tooth part. The drill holding part has a plurality of slits formed along an axial direction from an end of the stopper opposite to the drill blade entering depth regulating part, detachably engages to the flange and holds the drill by elasticity.

Further, in the stopper having such a configuration, the inventors also found out that it is preferable to prepare a plurality of stoppers having the drill blade entering depth regulating part with a different height as one set, for enabling to regulate for each entering depth of the blade of the drill in the jawbone of the lost tooth part.

The stopper attached to a drill having a flange projecting at a shank according to the present invention, its entirety is configured to be cylindrical shape, so that the stopper can be inserted from the top side of the blade of the drill. Further, the stopper has a through hole to be inserted by the blade of the drill, and includes the drill blade entering depth regulating part and the drill holding part. The drill blade entering depth regulating part has a height regulating the entering depth of the blade of the drill in the jawbone of the lost tooth part. The drill holding part has a plurality of the silts along the axial direction from the end opposite to the drill blade entering depth regulating part and can be detachably engaged to the flange of the drill by elasticity. Thus, the drill holding part having a plurality of the silts along the axial direction from the end opposite to the drill blade entering depth regulating part, detachably engages to the flange of the drill by elasticity, and holds the drill, so that the stopper can be detachably attached to the drill, regardless of the size of the diameter of the blade of the drill. Further, when the stopper is attached to the drill, the drill blade entering depth regulating part regulates the entering depth of the blade of the drill in the jawbone of the lost tooth part, so that the entering depth of the blade of the drill in the jawbone of the lost tooth part can be regulated simply and certainly.

Further, in the stopper having such a configuration, it is preferable to prepare a plurality of stoppers having different heights of the drill blade entering depth regulating part as one set, for enabling to regulate each depth of the blade of the drill entering in the jawbone of the lost tooth part.
Fig. 1 is a perspective view illustrating an example of the stopper according to the present invention.
Fig. 2 is a perspective view illustrating an example of drill having a flange projecting at a shank.
Fig. 3 is a perspective view illustrating a state in which the stopper illustrated in Fig. 1 is attached to the drill illustrated in Fig. 2.
Fig. 4 is a partial cross-sectional view illustrating a procedure in which the stopper illustrated in Fig. 1 is attached to the drill illustrated in Fig. 2.
Fig. 5 is a perspective view illustrating an example of a stopper set including a plurality of kinds of stoppers illustrated in Fig. 1, having different heights of the drill blade entering depth regulating part.

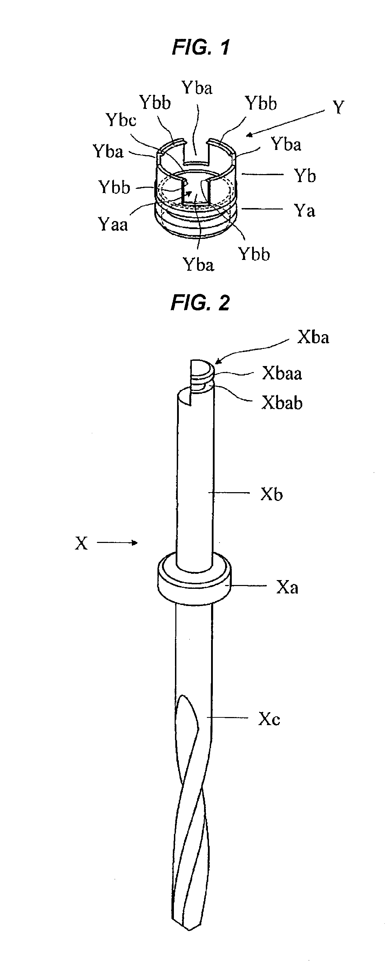

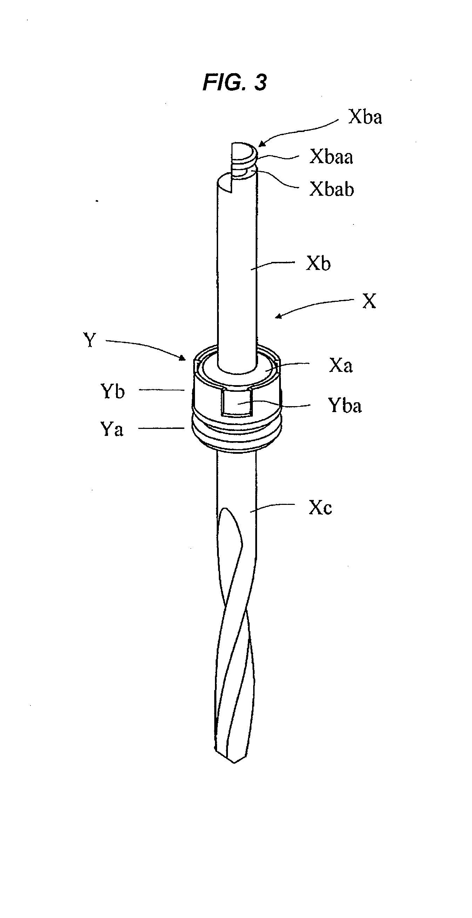

In figures, a drill X is attached to a dental driving apparatus such as dental hand piece and bores a dental implant fixture embedding hole in a jawbone of a lost tooth part. The drill X includes a shank Xb in which a flange Xa is projected and attached to a dental driving apparatus such as dental hand piece, and a blade Xc boring the jawbone. In addition, as illustrated in Fig. 2 and Fig. 3, at an opposite end of the blade side of the shank Xb of the drill X, a dental driving apparatus attachment part Xba is provided. The dental driving apparatus attachment part Xba includes a rotation transfer part Xbaa in which torque is transferred from dental driving apparatus such as a dental hand piece and a fall out prevention groove Xbab.

A stopper Y according to the present invention is configured with a cylindrical shape in its entirety and includes a drill blade entering depth regulating part Ya and a drill holding part Yb. The drill blade entering depth regulating part Ya regulates the entering depth of the blade Xc of the drill X and the drill holding part Yb engages to the flange Xa of the drill by elasticity to hold the drill. In addition, the stopper Y is made of a hard material, which can be sterilized, such as a stainless steel, or the like.

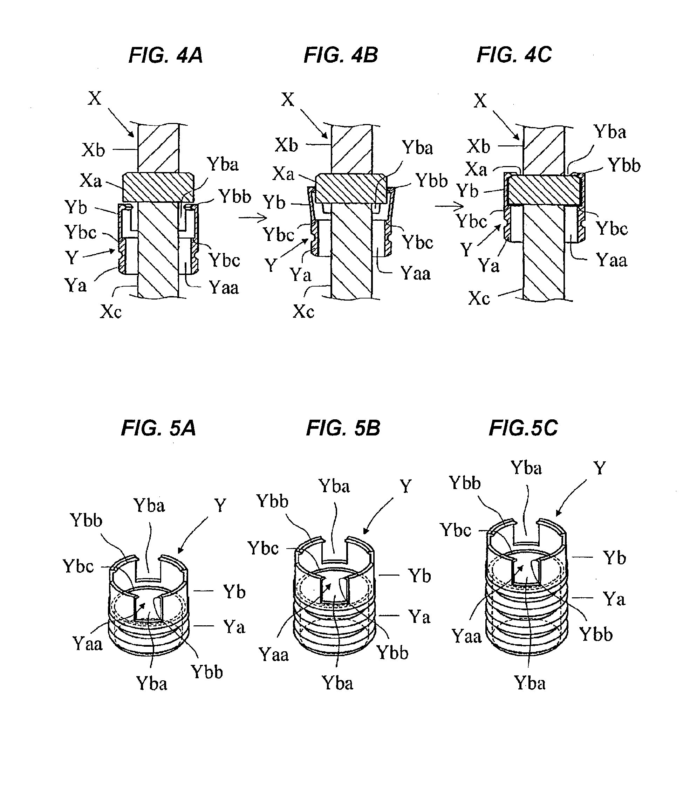

A drill entering depth regulating part Ya is a portion to regulate the entering depth of the blade Xc of the drill X in the jawbone of the lost tooth part and has a height to regulate the entering depth of the blade Xc of the drill X in the jawbone of the lost tooth part. When the stopper Y is attached to the drill X through the procedure which is illustrated in Figs. 4(a) to 4(c), the stopper can regulate the entering depth in the jawbone of the lost tooth part in a state that the inner peripheral surface of the drill blade entering depth regulating part Ya is not contacted to the blade Xc of the drill X. Thus, the diameter of a through hole Yaa is formed to be larger than the diameter of the blade Xc of the drill X and the blade Xc of the drill X is inserted in the through hole Yaa. In addition, as described later, the drill holding part Yb is detachably engaged to the flange Xa of the drill X and holds the drill X by elasticity, so that the stopper Y is attached to the drill X. Thus, the diameter of the through hole Yaa is formed to be smaller than the diameter of the flange Xa of the drill X and the inner diameter of the drill holding part Yb.

The drill holding part Yb is a portion to detachably engage to the flange Xa of the drill X by elasticity and holds the drill X. For enabling to attach/detach by elasticity, a plurality of slits Yba is formed along the axial direction from the end opposite to the drill blade entering depth regulating part Ya. Here, the meaning of "detachably engage by elasticity" is the state which is detachably engaged by using the elastic deformation of the drill holding part Yb without using connecting or fixing parts such as a screw, or the like. For example, as illustrated in Figs. 4(a) to 4(c), the drill holding part Yb, in which a protrusion edge Ybb is provided in the inner peripheral side adjacent the end opposite to the drill blade entering depth regulating part Ya, is expanded once and returns to the original state by the elastic deformation, so that the flange Xa of the drill X is pinched by the protrusion edge Ybb and a step part Ybc of the drill holding part Yb.

The stopper Y with such a configuration can be inserted from the top side of the blade Xc of the drill X since the entirety of the stopper is formed by a cylindrical shape. Further, the drill holding part Yb, which has a plurality of slits Yba formed along the axial direction from the end opposite to the drill blade entering depth regulating part Ya, detachably engages to the flange Xa of the drill X by elasticity, and holds the drill X, so that the stopper Y is detachably attached to the drill X. Further, when the stopper Y is attached to the drill X, the blade Xc inserts in the through hole Yaa without contacting to the inner peripheral surface of the drill blade entering depth regulating part Ya. Therefore, by unifying the diameter of the flange Xa of the drill, the stopper can be detachably attached to the drill X, regardless of the size of diameter of the blade Xc of the drill X. Furthermore, when the stopper Y is attached to the drill X, the drill blade entering depth regulating part Ya is arranged to regulate the entering depth in the jawbone of the lost tooth part, so that the entering depth of the blade Xc of the drill X in the jawbone of the lost tooth part can be easily and certainly regulated.

Further, as illustrated in Figs. 5(A) to 5(C), for regulating each entering depth of the blade Xc of the drill X in the jawbone of the lost tooth part, it is preferable to prepare a plurality of kinds of stoppers Y as one set, where the stoppers Y have a different height of the drill blade entering depth regulating part Ya. In this case, as for the height of the drill blade entering depth regulating part Ya, it is preferable that the height of the drill blade entering depth regulating part Ya can be easily recognized by the numbers of grooves provided on the outer peripheral surface of the drill blade entering depth regulating part Ya in parallel to the axial direction of the stopper Y.

## Claims

1. A stopper (Y) for regulating an entering depth of a blade (Xc) of a drill (X) in a jawbone of a lost tooth part and having a cylindrical shape,
wherein the drill (X) is for boring a dental implant fixture embedding hole in a jawbone of a lost tooth part and has a flange (Xa) projecting at a shank (Xb), wherein the stopper is inserted from a top side of the blade (Xc) of the drill (X) and detachably attached to the drill (X),
the stopper (Y) comprising:
a through hole (Yaa) being inserted by the blade (Xc) of the drill (X),
a drill blade entering depth regulating part (Ya) having a height for regulating an entering depth of the blade (Xc) of the drill (X) in a jawbone of a lost tooth part,
and
a drill holding part (Yb) for holding the drill (X),
wherein a plurality of slits (Yba) are formed along an axial direction from an end of the stopper opposite to the drill blade entering depth regulating part (Ya), and
wherein the drill holding part (Yb) detachably engages to the flange (Xa) of the drill (X) and holds the drill (X) by elasticity.

2. A stopper set comprising a plurality of stoppers according to claim 1, wherein the stoppers have the drill blade entering depth regulating parts (Ya) having a different height.
